# EUROPEAN PATENT SPECIFICATION

(11) **EP 0 389 925 B1**
(45) Date of publication and mention of the grant of the patent: **27.12.1995**
(21) Application number: 90105205.0
(22) Date of filing: 20.03.1990
(51) Int. Cl.: C07K 1/14

(54) **A method for purifying an outer membrane protein of Haemophilus influenzae**
Verfahren zur Reinigungbvon Aussenmembran-Protein von Haemophilus influenzae
Méthode de purification d'une protéine de membrane externe d'Haemophilus influenzae

(30) Priority: 29.03.1989 US 330229
(43) Date of publication of application: 03.10.1990
(73) Proprietor: THE RESEARCH FOUNDATION OF STATE UNIVERSITY OF NEW YORK, Albany, New York 12246 (US)
(72) Inventor: Murphy, Timothy F., Amherst, New York 14120 (US); Apicella, Michael A., Pendleton, New York 14120 (US)
(74) Representative: Liesegang, Roland, Dr.-Ing.

(56) References cited:
- WO-A-88/04932
- INFECTION AND IMMUNITY, vol. 54, no. 3, December 1986, pages 774-779, American Society for Microbiology; T.F. MURPHY et al.: "Antigenic characterization of the P6 protein of nontypable Haemophilus influenzae"

## Description

### BACKGROUND OF THE INVENTION

Haemophilus influenzae type b has long been recognized as a frequent pathogen, particularly in infants and children, but only recently has nontypable H. influenzae been recognized as an important pathogen. It is now well established that nontypable H. influenzae causes pneumonia, bacteremia, meningitis, postpartum sepsis, and acute febrile tracheobronchitis in adults. In addition, nontypable H. influenzae causes neonatal sepsis and is a frequent etiologic agent in acute otitis media in infants and children. Therefore, the importance of discovering a method to assay a clinical sample such as sputum, cerebral spinal fluid, blood and others for the presence of H. influenzae is clear.

The observation that nontypable H. influenzae causes serious infections in adults and children has stimulated interest in study of the pathogenesis and potential virulence factors associated with this bacterium. The ribitol capsule of H. influenzae type b is a virulence factor for the organism, and antibody to capsule protects the host by means of bactericidal and/or opsonizing actions. These observations have generated much investigation on the role of the capsular polysaccharide in infection with H. influenzae type b and protection from these infections. However, nontypable H. influenzae lacks a polysaccharide capsule, and, similar to the outer membranes of other gram-negative bacteria, the outer membrane of H. influenzae is composed of outer membrane proteins (OMPs) and lipopolysaccharide (LPS). Therefore, studies of the relationship between virulence of nontypable H. influenzae and surface antigens focus on OMPs and LPS.

Analysis of OMPs of nontypable H. influenzae has shown that there are marked differences in OMP composition among strains. See e.g. Murphy et at, "A Subtyping System For Nontypable Haemophilus influenzae Based on Outer-membrane Proteins," J. Infect. Dis, 1983, 147:838-46; Barenkamp et al, "Outer Membrane Protein and Biotype Analysis of Pathogenic Nontypable Haemophilus influenzae," Infect. Immun, 1982, 36:535-40; Lorb et al, "Outer Membrane Protein Composition in Disease Isolates of Haemophilus influenzae, Pathogenic and Epidemiological Implications," Infect. Immun, 1980, 30:709-17.

A subtyping system for nontypable H. influenzae based on the major OMPs has previously been developed. A surface exposed antigen (immunogen) which is conserved in all strains is an important tool in developing a method of identifying H. influenzae in clinical specimens as well as a vaccine against H. influenzae.

A relatively simple method for purifying the conserved surface exposed antigen would be desirable. Methods that have been used included the step of initially isolating the outer membrane and the resultant products contained contaminating RNA, other cellular components and detergents.

Illustrative for such more complicated and/or less effective processes are the methods as described in WO 88/04932 and Infection and Immunity 54/3 (1986), 744-9.

It is therefore an object of this invention to provide a method for purifying the conserved surface exposed antigen, which is relatively simple and produces an increased yield of the desired antigen. It is a further object of this invention to provide a purification method which will produce a product free of contaminating RNA and undesirable cellular components.

### BRIEF DESCRIPTION OF THE INVENTION

In accordance with the present invention there is provided a method for purifying an immunogenic outer membrane protein of H. influenzae (H. flu) consisting essentially of steps (a) to (f) of claim 1.

### DETAILED DESCRIPTION OF THE INVENTION

A method for purifying the outer membrane protein of Haemophilus influenzae is disclosed in this invention.

The method disclosed herein is relatively simpler than previous methods. The method utilizes the whole bacterium as a starting material for incubation in detergent containing buffers, therefore eliminating the initial step of isolating the outer membrane, as was necessary in previous methods. By whole bacterium is meant that the bacterium is used in an unprocessed state.

H. flu, in a suspending medium, is used to directly obtain a first fraction comprising the outer membrane protein associated with peptidoglycan. The suspending medium is a liquid in which the fraction is insoluble and may be a detergent buffer. By detergent buffer is meant a buffer solution comprising a detergent wherein the outer membrane protein and the peptidoglycan are insoluble. Suitable detergents include sodium dodecyl sulfate (SDS). An example of a suitable detergent buffer is Buffer B. Buffer B comprises 1% SDS, 0.1% Beta-mercaptoethanol, 0.01M tris, 0.5M NaCl, pH 8.0. The first fraction comprising the outer membrane protein (p6) associated with peptidoglycan is obtained by successively heat treating and centrifuging the suspension. The suspension may be sonicated briefly to help suspend the cells. Heat treatment may be by any suitable means known to those skilled in the art. An example of a suitable means for heat treatment is incubation at between about 20°C to about 37°C for between about 20 to 40 minutes. Centrifugation is at about 20,000g to about 40,000g for about 30-60 minutes at ambient temperature. Ambient temperature as used herein means between about 20°C to about 27°C. By successive heat treatment and centrifugation is meant that the H. flu, in the suspending medium, is heat treated then centrifuged then resuspended in the suspending medium and heat treated and centrifuged until the first fraction comprising the p6 associated with peptidoglycan is formed.

Contaminating RNA is then digested from the first fraction by use of a digesting material in a first liquid in which the peptidoglycan associated with the outer membrane protein remains insoluble and the digested contaminating RNA is soluble, such that a second fraction is formed. The second fraction is insoluble in the first liquid and comprises the outer membrane protein associated with peptidoglycan. By contaminating RNA is meant RNA which is present in the second fraction along with p6 and peptidoglycan. A digesting material allows for the digestion of contaminating RNA while the p6 remains in an insoluble form by virtue of its association with peptidoglycan. An example of a digesting material is ribonuclease A. The first liquid may be a detergent buffer as discussed supra. By soluble is meant selectively suspendable or removable with respect to the second fraction. Digesting material is added to the detergent buffer and the mixture is sequentially heat treated and centrifuged. Heat treatment and centrifuging are as described supra. By sequential heat treatment and centrifuging is meant that the first fraction is suspended in the detergent buffer comprising the digesting material and heat treated and centrifuged until contaminating RNA is digested and the second fraction is formed.

The outer membrane protein (p6) is separated from the second fraction by suspending in a second liquid in which said outer membrane protein is soluble and the peptidoglycan is insoluble and heat treating to release the outer membrane protein from the peptidoglycan to obtain a third liquid comprising the solubilized outer membrane protein. An example of a liquid in which said outer membrane protein is soluble and the peptidoglycan is insoluble is a detergent free buffer. By detergent free buffer is meant a buffer solution not having a detergent added. Examples of suitable detergent free buffer solutions are tris buffer, PBS and sodium tetraborate buffer. A preferred detergent free buffer is sodium tetraborate buffer. The second fraction is suspended in the detergent free buffer and heat treated at between about 50-75°C for about 30-60 minutes. The suspension is then centrifuged at about 80,000g to about 120,000g for a sufficient time at a sufficient temperature until a supernatant (third liquid) comprising the outer membrane protein is obtained. The supernatant is then recovered which comprises a soluble preparation of p6 which is free of undesirable cellular components. Undesirable cellular components include lipooligosaccharides, proteins other than p6, peptidoglycan, DNA and RNA.

The supernatant is then concentrated by means known to those skilled in the art and includes the method of pressure filtration.

The following example describes the manner and process of making and using the invention and set forth the best mode contemplated by the inventor of carrying out the invention, but is not to be construed as limiting.

Bacteria were grown in broth and 25 grams of bacteria were suspended in Buffer B, sonicated briefly to help suspend the cells and incubated at 37°C for 30 minutes. After incubation the suspension was centrifuged at 20,000g for 30 minutes at 25°C. The supernatant was discarded and the insoluble fraction was resuspended in Buffer B, incubated, and centrifuged as before. This cycle of incubations and centrifugations was repeated a total of 5 times. For the last two incubations, ribonuclease A (10 micrograms/ml) is added to Buffer B. Including the ribonuclease at this step allows for digestion of contaminating RNA while the p6 remains in an insoluble form by virtue of its association with peptidoglycan. In this way, the ribonuclease and the RNA degradation products remain soluble while p6 and peptidoglycan remain insoluble.

The p6/peptidoglycan insoluble fraction was suspended in 0.1M Sodium tetraborate buffer (pH 9.5) and incubated for 30 minutes at 65° C. The mixture was then centrifuged at 100,000 g for one hour at 35° C. This step releases p6 from peptidoglycan so that solubilized, p6 remains in the supernatant. This supernatant was then concentrated by pressure filtration approximately ten fold. The volume was brought back up to its original volume and reconcentrated. This concentrating and washing procedure was done five times using the borate buffer. The resulting p6 preparations have been characterized as follows: SDS-PAGE yields a single protein band at a molecular weight of approximately 16,000 daltons. The preparation is free of detectable lipooligosaccharide using a monoclonal antibody. An absorbance spectrum of the soluble p6 revealed a peak at a wavelength of 275 nM. Agarose-gel electrophoresis revealed that the preparation is free of DNA and RNA. Therefore, the final preparation contains purified, solubilized p6 protein from Haemophilus influenzae. Amino acid analysis of this material shows that the amino acid content is consistent with that predicted from the DNA sequence disclosed herein.

Other embodiments of the invention will be apparent to the skilled in the art from a consideration of this specification or practice of the invention disclosed herein. It should be understood that there may be other embodiments which fall within the spirit and scope of the invention as defined by the following claims.

## Claims

1. A method for purifying an immunogenic outer membrane protein of Haemophilus influenzae consisting essentially of:
a) suspending H. influenzae micro-organisms by incubating the organisms in a detergent buffer solution to form an insoluble fraction comprising the outer membrane protein and peptidoglycan component and a soluble fraction comprising the remainder of the cellular components;
b) separating the insoluble fraction of step a) from the soluble fraction;
c) suspending the insoluble fraction of step b) in detergent buffer containing digesting material that allows for digestion of contaminating RNA and allowing for RNA digestion;
d) separating the insoluble fraction of step c) from the soluble fraction comprising the digesting material and digested RNA;
e) solubilizing the insoluble fraction of step d) by heat-treating in a detergent-free buffer; and
f) separating the soluble fraction containing the purified outer membrane protein from the insoluble fraction of step e) containing the peptidoglycan component.

2. The method of claim 1, wherein the purified outer membrane protein is P6 having a molecular weight by SDS-PAGE under non-reducing conditions of from about 15,000 to about 17,000 daltons.

3. The method of claim 1 or 2, wherein said detergent buffer solution of step a) comprises an anionic detergent.

4. The method of claim 3, wherein said anionic detergent is sodium dodecyl sulfate.

5. The method of one of claim 1 to 4, wherein said buffer solution is buffer B comprising 1 wt. % sodium dodecyl sulfate, 0.01 M Tris, 0.5 M NaCl, 0.1 % by volume beta-mercaptoethanol, pH 8.0.

6. The method of one of claim 1 to 5, wherein step a) further comprises sonication of the micro-organisms in the detergent buffer solution to facilitate suspension of the cellular components.

7. The method of one of claims 1 to 2, wherein said incubating of step a) is conducted by heat-treating the suspension at about 37°C for about 30 minutes.

8. The method of one of claims 1 to 7, wherein the insoluble fraction is separated from the soluble fraction in step b) by centrifugation at about 100,000 xg for about 60 minutes, the insoluble fraction being in the form of a pellet.

9. The method of claim 8, further including the steps of:
resuspending the insoluble fraction in a buffer solution; and
repeating the heat treating of step a) and b) until the insoluble fraction is substantially free of soluble cellular components.

10. The method of one of claims 1 to 9, wherein said RNA digestion occurs by incubating the solution at about 37°C, for about 30 minutes.

11. The method of one of claims 1 to 10, wherein the digesting material and digested RNA are separated out of the insoluble fraction in step d) by centrifugation.

12. The method of one of claims 1 to 11, wherein said detergent-free buffer comprises 0.1 M sodium tetraborate, pH 9.5.

13. The method of one of claims 1 to 12, wherein step f) further comprises:
incubating the insoluble fraction at about 65°C for about 30 minutes;
immediately thereafter centrifuging the insoluble fraction at about 100,000 xg for about 1 hour at about 37°C; and
collecting the soluble fraction which contains the outer membrane protein.

14. The method of claim 13, wherein the soluble fraction is treated with pressure filtration to concentrate the outer membrane protein.

15. A method for purifying an outer membrane protein of Haemophilus influenzae consisting essentially of:
a) suspending H. influenzae micro-organisms in a detergent buffer solution to form a suspension;
b) heat-treating the suspension by incubating at about 37°C for about 30 minutes to form a soluble and insoluble fraction;
c) separating the insoluble fraction from the soluble fraction by centrifuging the suspension of step b) at about 100,000 xg for about 60 minutes at ambient temperature, the insoluble fraction being in the form of a pellet comprising the outer membrane protein and peptidoglycan component and the soluble fraction comprising the remainder of the cellular components;
d) resuspending the pellet of step c) in a detergent buffer solution;
e) repeating steps b) and c) until the insoluble fraction is substantially free of soluble cellular components;
f) digesting RNA present in the insoluble fraction by adding ribonuclease A and incubating at about 37°C for about 30 minutes;
g) repeating step c) to remove the ribonuclease A which will be contained in the soluble fraction;
h) solubilizing the insoluble fraction of step g) in a detergent-free buffer solution comprising 0.1 M sodium tetraborate, pH 9.5 by incubating at about 65°C for about 30 minutes; and
i) separating the outer membrane protein from the peptidoglycan component by centrifuging the solution of h) at about 100,000 xg, for about 1 hour at about 37°C, wherein the supernatant comprises the outer membrane protein.

## Patentansprüche

1. Ein Verfahren zur Reinigung eines immunogenen Außenmembran-Proteins von Haemophilus influenzae, im wesentlichen bestehend aus:
a) Suspendieren von H. influenzae-Mikroorganismen durch Inkubieren der Organismen in einer Detergenspufferlösung, um eine unlösliche Fraktion zu bilden, die das Außenmembran-Protein und die Peptidoglycan-Komponente umfaßt, und eine lösliche Fraktion, die den Rest der zellulären Komponenten umfaßt;
b) Trennen der unlöslichen Fraktion aus Schritt a) von der löslichen Fraktion;
c) Suspendieren der unlöslichen Fraktion aus Schritt b) in Detergenspuffer, der Verdauungsmaterial enthält, das Verdauung kontaminierender RNA ermöglicht, und Ermöglichen von RNA-Verdauung;
d) Trennen der unlöslichen Fraktion aus Schritt c) von der löslichen Fraktion, die das Verdauungsmaterial und die verdaute RNA umfaßt;
e) Solubilisieren der unlöslichen Fraktion aus Schritt d) durch Hitzebehandlung in einem Detergens-freien Puffer; und
f) Trennen der löslichen Fraktion, die das gereinigte Außenmembran-Protein der äußeren Membran enthält, von der unlöslichen Fraktion aus Schritt e), die die Peptidoglycan-Komponente enthält.

2. Das Verfahren nach Anspruch 1, wobei das gereinigte Protein der äußeren Membran P6 ist, das nach SDS-PAGE unter nicht-reduzierenden Bedingungen ein Molekulargewicht von etwa 15.000 bis 17.000 Dalton aufweist.

3. Das Verfahren nach Anspruch 1 oder 2, wobei besagte Detergenspufferlösung aus Schritt a) ein anionisches Detergens umfaßt.

4. Das Verfahren nach Anspruch 3, wobei besagtes anionische Detergens Natriumdodecylsulfat ist.

5. Das Verfahren nach einem der Ansprüche 1 bis 4, wobei besagte Pufferlösung Puffer B ist, der 1 Gew.-% Natriumdodecylsulfat, 0,01 M Tris, 0,5 M NaCl, 0,1 Vol.-% beta-Mercaptoethanol, pH 8,0, umfaßt.

6. Das Verfahren nach einem der Ansprüche 1 bis 5, wobei Schritt a) weiterhin Beschallung der Mikroorganismen in der Detergenspufferlösung umfaßt, um Suspendierung der zellulären Komponenten zu erleichtern.

7. Das Verfahren nach einem der Ansprüche 1 bis 2, wobei besagtes Inkubieren bei Schritt a) durch Hitzebehandeln der Suspension bei etwa 37°C für etwa 30 Minuten ausgeführt wird.

8. Das Verfahren nach einem der Ansprüche 1 bis 7, wobei die unlösliche Fraktion von der löslichen Fraktion in Schritt b) durch Zentrifugation bei etwa 100.000 xg für etwa 60 Minuten getrennt wird, wobei die unlösliche Fraktion in der Form eines Pellets vorliegt.

9. Das Verfahren nach Anspruch 8, das weiterhin die Schritte umfaßt:
Resuspendieren der unlöslichen Fraktion in einer Pufferlösung; und
Wiederholen der Hitzebehandlung von Schritt a) und b) bis die unlösliche Fraktion im wesentlichen frei von löslichen zellulären Komponenten ist.

10. Das Verfahren nach einem der Ansprüche 1 bis 9, wobei besagte RNA-Verdauung dadurch vorgenommen wird, daß die Lösung bei etwa 37°C für etwa 30 Minuten inkubiert wird.

11. Das Verfahren nach einem der Ansprüche 1 bis 10, wobei das Verdauungsmaterial und die verdaute RNA aus der unlöslichen Fraktion in Schritt d) durch Zentrifugation getrennt werden.

12. Das Verfahren nach einem der Ansprüche 1 bis 11, wobei besagter Detergens-freier Puffer 0,1 M Natriumtetraborat, pH 9,5, umfaßt.

13. Das Verfahren nach einem der Ansprüche 1 bis 12, wobei Schritt f) weiterhin umfaßt:
Inkubieren der unlöslichen Fraktion bei etwa 65°C für etwa 30 Minuten;
unmittelbar danach Zentrifugieren der unlöslichen Fraktion bei etwa 100.000 xg für etwa 1 Stunde bei etwa 37°C; und
Sammeln der löslichen Fraktion, die das Außenmembran-Protein enthält.

14. Das Verfahren nach Anspruch 13, wobei die lösliche Fraktion mit Druckfiltration behandelt wird, um das Außenmembran-Protein zu konzentrieren.

15. Ein Verfahren zum Reinigen eines Außenmembran-Proteins von Haemophilus influenzae, im wesentlichen bestehend aus:
a) Suspendieren von H. influenzae-Mikroorganismen in einer Detergenspufferlösung, um eine Suspension zu bilden;
b) Hitzebehandeln der Suspension durch Inkubation bei etwa 37°C für etwa 30 Minuten, um eine lösliche und eine unlösliche Fraktion zu bilden;
c) Trennen der unlöslichen Fraktion von der löslichen Fraktion durch Zentrifugieren der Suspension aus Schritt b) bei etwa 100.000 xg für etwa 60 Minuten bei Umgebungstemperatur, wobei die unlösliche Fraktion in der Form eines Pellets vorliegt, das das Außenmembran-Protein und die Peptidoglycan-Komponente umfaßt, und die lösliche Fraktion, die den Rest der zellulären Komponenten umfaßt;
d) Resuspendieren des Pellets aus Schritt c) in einer Detergenspufferlösung;
e) Wiederholen der Schritte b) und c), bis die unlösliche Fraktion im wesentlichen frei von löslichen zellulären Komponenten ist;
f) Verdauen von RNA, die in der unlöslichen Fraktion vorhanden ist, durch Zugabe von Ribonuclease A und Inkubieren bei etwa 37°C für etwa 30 Minuten;
g) Wiederholen von Schritt c), um die Ribonuclease A zu entfernen, die in der löslichen Fraktion enthalten sein wird;
h) Solubilisieren der unlöslichen Fraktion aus Schritt g) in einer Detergens-freien Pufferlösung, die 0,1 M Natriumtetraborat, pH 9,5, umfaßt, durch Inkubieren bei etwa 65°C für etwa 30 Minuten; und
i) Trennen des Außenmembran-Proteins von der Peptidoglycan-Komponente durch Zentrifugieren der Lösung aus h) bei etwa 100.000 xg für etwa 1 Stunde bei etwa 37°C, wobei der Überstand das Außenmembran-Protein.

## Revendications

1. Un procédé de purification d'une protéine immunogène de membrane externe de *Haemophilus influenzae*, consistant essentiellement à :
a) mettre en suspension des microorganismes *H. influenzae* par incubation des microorganismes dans une solution tampon avec détergent pour former une fraction insoluble comprenant la protéine de membrane externe et le constituant peptidoglycane et une fraction soluble comprenant le reste des constituants cellulaires ;
b) séparer la fraction insoluble de l'étape a) d'avec la fraction soluble ;
c) mettre en suspension la fraction insoluble de l'étape b) dans du tampon avec détergent contenant une substance de digestion qui permet de digérer l'ARN contaminant, et permettre la digestion de l'ARN ;
d) séparer la fraction insoluble de l'étape c) d'avec la fraction soluble comprenant la substance de digestion et l'ARN digéré ;
e) solubiliser la fraction insoluble de l'étape d) par traitement thermique dans un tampon sans détergent ; et
f) séparer la fraction soluble contenant la protéine de membrane externe purifiée d'avec la fraction insoluble de l'étape e) contenant le constituant peptidoglycane.

2. Le procédé de la revendication 1, dans lequel la protéine de membrane externe purifiée est P6 ayant un poids moléculaire par EGPA-DSS en conditions non réductrices d'environ 15 000 à environ 17 000 daltons.

3. Le procédé de la revendication 1 ou 2, dans lequel ladite solution tampon avec détergent de l'étape a) comprend un détergent anionique.

4. Le procédé de la revendication 3, dans lequel ledit détergent anionique est le dodécylsulfate de sodium.

5. Le procédé de l'une des revendications 1 à 4, dans lequel ladite solution tampon est le tampon B comprenant 1 % en poids de dodécylsulfate de sodium, Tris 0,01M, NaCl 0,5M, 0,1 % en volume de bêta-mercaptoéthanol, pH 8,0.

6. Le procédé de l'une quelconque des revendications 1 à 5, dans lequel l'étape a) consiste de plus à traiter aux ultrasons les microorganismes dans la solution tampon avec détergent pour faciliter la mise en suspension des constituants cellulaires.

7. Le procédé de l'une des revendications 1 et 2, dans lequel ladite incubation de l'étape a) est conduite par traitement thermique de la suspension à environ 37°C pendant environ 30 minutes.

8. Le procédé de l'une des revendications 1 à 7, dans lequel la fraction insoluble est séparée de la fraction soluble dans l'étape b) par centrifugation à environ 100 000 *g* pendant environ 60 minutes, la fraction insoluble étant sous la forme d'un culot.

9. Le procédé de la revendication 8, comprenant de plus les étapes suivantes :
remettre en suspension la fraction insoluble dans une solution tampon ; et
répéter le traitement thermique de l'étape a) et b) jusqu'à ce que la fraction insoluble soit sensiblement exempte de constituants cellulaires solubles.

10. Le procédé de l'une des revendications 1 à 9, dans lequel ladite digestion d'ARN s'effectue par incubation de la solution environ à 37°C pendant environ 30 minutes.

11. Le procédé de l'une des revendications 1 à 10, dans lequel la substance de digestion et l'ARN digéré sont séparés de la fraction insoluble dans l'étape d) par centrifugation.

12. Le procédé de l'une des revendications 1 à 11, dans lequel ledit tampon sans détergent comprend du tétraborate de sodium 0,1M, pH 9,5.

13. Le procédé de l'une des revendications 1 à 12, dans lequel l'étape f) consiste de plus à :
faire incuber la fraction insoluble à environ 65°C pendant environ 30 minutes ;
immédiatement après, centrifuger la fraction insoluble à environ 100 000 *g* pendant environ 1 heure à environ 37°C ; et
recueillir la fraction soluble qui contient la protéine de membrane externe.

14. Le procédé de la revendication 13, dans lequel la fraction soluble est traitée par filtration sous pression pour concentrer la protéine de membrane externe.

15. Un procédé de purification d'une protéine de membrane externe de *Haemophilus influenzae*, consistant essentiellement à :
a) mettre en suspension des microorganismes *H. influenzae* dans une solution tampon avec détergent pour former une suspension ;
b) traiter thermiquement la suspension par incubation à environ 37°C pendant environ 30 minutes pour former une fraction soluble et une fraction insoluble ;
c) séparer la fraction insoluble de la fraction soluble en centrifugeant la suspension de l'étape b) à environ 100 000 *g* pendant environ 60 minutes à la température ambiante, la fraction insoluble étant sous la forme d'un culot comprenant la protéine de membrane externe et le constituant peptidoglycane et la fraction soluble comprenant le reste des constituants cellulaires ;
d) remettre en suspension le culot de l'étape c) dans une solution tampon avec détergent ;
e) répéter les étapes b) et c) jusqu'à ce que la fraction insoluble soit sensiblement exempte de constituants cellulaires solubles ;
f) digérer l'ARN présent dans la fraction insoluble en ajoutant de la ribonucléase A et en faisant incuber à environ 37°C pendant environ 30 minutes ;
g) répéter l'étape c) pour éliminer la ribonucléase A qui est contenue dans la fraction soluble ;
h) solubiliser la fraction insoluble de l'étape g) dans une solution tampon sans détergent comprenant du tétraborate de sodium 0,1M, pH 9,5, par incubation à environ 65°C pendant environ 30 minutes ; et
i) séparer la protéine de membrane externe du constituant peptidoglycane en centrifugeant la solution de h) à environ 100 000 *g* pendant environ 1 heure à environ 37°C, le surnageant comprenant la protéine de membrane externe.
